# EUROPEAN PATENT SPECIFICATION

(11) **EP 2 699 251 B1**
(45) Date of publication and mention of the grant of the patent: **22.03.2017**
(21) Application number: 12714027.5
(22) Date of filing: 17.04.2012
(51) Int. Cl.: A61K 45/06, A61K 38/01, A23L 33/105, A23L 33/115, A23L 33/15, A23L 33/155, A23L 33/16, A23L 33/18, A23L 33/19, A61P 9/00

(54) **INFANT FORMULA FOR USE IN THE PREVENTION OF CARDIOVASCULAR DISEASES**
SÄUGLINGSNAHRUNG ZUR VERWENDUNG BEI DER PRÄVENTION VON KARDIOVASKULÄREN ERKRANKUNGEN
PRÉPARATION POUR NOURRISSONS DESTINÉE À ÊTRE UTILISÉE DANS LA PRÉVENTION DE MALADIES CARDIO-VASCULAIRES

(30) Priority: 19.04.2011 EP 11163011
(43) Date of publication of application: 26.02.2014
(73) Proprietor: Nestec S.A., 1800 Vevey (CH)
(72) Inventor: MACE, Catherine, CH-1005 Lausanne (CH); STEENHOUT, Philippe, CH-1814 La Tour-de-Peilz (CH)
(74) Representative: Corticchiato, Olivier
(86) International application number: PCT/EP2012/057024
(87) International publication number: WO 2012/143362

(56) References cited:
- EP-A1- 1 932 437
- WO-A1-2006/069918
- WO-A2-2007/004878
- WO-A2-2010/070613
- KOLETZKO B ET AL: "[Infant feeding practice and later obesity risk. Indications for early metabolic programming].", BUNDESGESUNDHEITSBLATT, GESUNDHEITSFORSCHUNG, GESUNDHEITSSCHUTZ JUL 2010 LNKD- PUBMED:20652481, vol. 53, no. 7, July 2010 (2010-07), pages 666-673, XP002653102, ISSN: 1437-1588

## Description

### Field of the invention

The present invention relates to an infant formula, preferably a starter infant formula, for use in preventing cardiovascular diseases and for use in preventing high blood pressure, especially later in life.

### Background of the invention

Blood pressure is the force of blood pushing against the walls of the arteries as the heart pumps out blood. If this pressure rises and stays high over time, it can damage the body in many ways.

Blood pressure numbers include systolic and diastolic pressures. Systolic blood pressure is the pressure when the heart beats while pumping blood. Diastolic blood pressure is the pressure when the heart is at rest between beats.

High blood pressure is a serious condition that is associated to higher risk of cardiovascular diseases, and can lead for example to coronary heart disease, heart failure, stroke, kidney failure, and other health problems. Among the cardiovascular diseases, the ones which present high blood pressure as one of their symptoms or causes are of particular interest.

Hypertension is a disease condition which is caused by a sustained high blood pressure. Hypertension is a cardiac chronic medical condition in which the systemic arterial blood pressure is outside a normal range. The hypertension range is generally referring to a condition where a systolic blood pressure is 140 mmHg or higher or a diastolic blood pressure is 90 mmHg or higher. Hypertension is classified as either primary (essential) or secondary. About 90-95% of cases are termed "primary hypertension", which refers to high blood pressure for which no medical cause has been found. The remaining 5-10% of cases (Secondary hypertension) are caused by other conditions that affect the kidneys, arteries, heart, or endocrine system.

The incidence of hypertension is increasing all over the world. In addition, hypertension may cause fatal complications such as cerebral stroke, heart failure, and coronary artery diseases, even among minor or mild patients exhibiting no external symptoms.

Some factors are associated with high blood pressure, including body mass and diet in adulthood, but environmental influences acting much earlier in life affect future blood pressure, like infant nutrition. For instance, Boubred et al. (2007) have shown that early postnatal overfeeding in the rat enhances postnatal nephrogenesis, but elevated blood pressure and glomerulosclerosis are still observed in male adults. Jarvisalo et al. (2009) have concluded that adult men who have been breastfed have better brachial endothelial function compared to men who have been formula fed.

Human breast milk represents the uncontested gold standard in terms of infant nutrition. However, in some cases breastfeeding is inadequate or unsuccessful for medical reasons or because of mother choice not to breastfeed. Infant formulae have been developed for these situations.

WO 2010/070613 A2 relates to a baby feeding formula and associated system. More specifically, it discloses infant formula with low protein content (below 1 g per 100mL) and low energy content (below 50 kcal per 100mL) for premature infants. No data is provided in relation with blood pressure.

WO 2007/004878 A2 relates to a method to treat and/or prevent childhood obesity which comprises administering a nutritional composition containing fat, digestivble carbohydrates and protein, where the protein comprises at least 25 % by weight of peptides with a chain length of 25 to 30 amino acids based on dry weight of proteins. It discloses a lower insulin secretion.

WO 2006/069918 A1 relates to the use of an infant formula with reduced protein content, to continuously reduce the circulating level of IGF-1 in the first few months of life of an infant.

EP 1932437 A1 relates to an infant formula for infants at risk of developing obesity later in life, with an energy density below 650 kcal per liter and a protein content below 1.8 g per 100 kcal. It does not discuss blood pressure.

In a review article entitled "Frühkindliche Ernährung und späteres Adipositasrisiko" (Bundesgesundheitsbl. 2010,53:666-673) Koletzko et al. discuss the potential benefit of a low protein formula for decreasing the risk of obesity.

Therefore, there is a need for an infant formula which may be used in the prevention of cardiovascular diseases or high blood pressure, generally in later life.

### Summary of the invention

The present invention in defined in the claim.

Accordingly, the present invention provides an infant formula according to the claims comprising at least a source of proteins, a source of lipids, a source of carbohydrates, wherein the protein content is within the range of 1.6 to 1,85g/100kcal, for use in the prevention of cardiovascular diseases or high blood pressure, especially later in life.

Another aspect of then present disclosure is a method for preventing cardiovascular diseases in a patient, comprising feeding said patient with an infant formula according to the claims.

Yet another aspect of the present disclosure is a method for preventing high blood pressure in a patient, comprising feeding said patient with an infant formula according to the claims.

The inventors have discovered that, surprisingly, infant formulas used according to the invention, are particularly advantageous for the prevention of high blood pressure, and potentially of cardiovascular diseases, in particular those cardiovascular diseases associated with high blood pressure. This encompasses coronary heart disease, heart failure, peripheral arterial disease, hypertensive retinopathy, hypertensive encephalopathy, stroke and kidney failure. The preventive effect can be observed in patients later in life, for instance as soon as 3 years old, although the infant formula was fed to the same patients only as an infant.

### Detailed description of the invention

As used herein, the following terms have the following meanings.

The term "infant" means a child under the age of 12 months.

The term "infant formula" means a foodstuff intended for particular nutritional use by infants during the first year of life and satisfying by itself the nutritional requirements of this category of person, as defined in European Commission Directive 91/321/EEC of May 14, 1991. The term "infant formula" includes hypoallergenic infant formulas. The term "infant formula" includes starter infant formula and follow-on formula.

The term "starter infant formula" means a foodstuff intended for particular nutritional use by infants during the first four to six months of life.

The term "follow-on formula" means a foodstuff intended for particular nutritional use by infants aged from four to six months, up to 12 months, and constituting the principal liquid element in the progressively diversified diet of this category of person.

The term "prevention of cardiovascular diseases" means the prevention and the reduction of frequency and/or occurrence and/or severity and/or duration of cardiovascular diseases, usually in later life. Occurrence is related to the number of any cardiovascular disease. Risk is related to the probability of appearance of cardiovascular diseases. Frequency is related to the number of the same cardiovascular diseases. Duration is related to the total duration of the cardiovascular diseases. Cardiovascular diseases are in particular those diseases associated with high blood pressure. Cardiovascular diseases include, for instance, coronary heart disease, heart failure, peripheral arterial disease, hypertensive retinopathy, hypertensive encephalopathy, stroke and kidney failure. On a population basis, it is estimated that a reduction of 2 mm Hg in diastolic blood pressure would result in a 15% reduction in risk of stroke and a 6% reduction in risk of coronary heart disease (Cook NR, Cohen J, Hebert P, Taylor JO, Hennekens CH. Implications of small reductions in diastolic blood pressure for primary prevention. Arch Intern Med.. 1995;155:701-709).

The term "later in life" encompasses the effect after the termination of the intervention. The effect "later in life" can be months or years after the termination of said intervention. Thus "later life" can mean at 3 years old, during the teenage period, or during the adult life. In the case of young children, this relates to an age of 2,5 years to 10 years. In the case of teenagers, it relates to an age of 10 to 20 years. In the case of adults, it relates to an age above 18 years. More generally, the duration of the use of the infant formula according to the invention has effect on the meaning of "later life".

All percentages are by weight unless otherwise stated.

According to the invention, the infant formula is preferably for use in the prevention of cardiovascular diseases associated with high blood pressure. In other words, high blood pressure can be one of the symptoms or the causes of said cardiovascular diseases.

Preferably, the protein content of the formula is within the range of 1.6 to 2.1g/100kcal. Preferably, the protein content is below 2.0g/100kcal, more preferably below 1.9g/100kcal, and even more preferably below 1.85g/100kcal. For instance, the protein content is within the range of 1.6 to 2.0g/100kcal, preferably within the range of 1.6 to 1.9g/100kcal, more preferably within the range of 1.6 to 1.85g/100kcal.

Milk proteins may be sourced from cow milk, goat milk, ewe milk, buffalo milk, camel milk, mare milk, and mixtures thereof.

Protein sources are preferably chosen within milk proteins, milk protein partial hydrolysates, hypoallergenic milk protein hydrolysates, and mixtures thereof. The proteins may be intact or hydrolysed or a mixture of intact and hydrolysed proteins. Preferably, an embodiment of the infant formula comprises whey proteins which are non-hydrolyzed.

In an embodiment, protein sources of the infant formula can be based on whey, casein and mixtures thereof, as well as protein sources based on soy. Thus the source of proteins comprises preferably whey proteins, casein or mixtures of both, and more preferably the source of proteins is exclusively whey proteins, casein or mixtures of both. In a preferred embodiment, the source of proteins is a mixture of whey proteins and casein, the whey/casein ratio being at least equal to 50/50, preferably at least equal to 60/40, more preferably at least equal to 70/30.

As far as whey proteins are concerned, the protein source may be based on acid whey or sweet whey or mixtures thereof and may include alpha-lactalbumin and beta-lactoglobulin in any desired proportions. When the infant formula comprises whey proteins, the major part of caseino-glyco-macropeptide (CGMP) has preferably been removed from whey proteins, that is to say that the content of CGMP is low. Preferably, the CGMP content is reduced by at least 80% with respect of the native whey protein. For instance, the protein source can comprise a hydrolysed sweet whey fraction from which over 85% of the CGMP originally present has been removed, as for example disclosed in example 1 of EP1220620B1.

It may be desirable to supply partially hydrolysed proteins, for example to infants believed to be at risk of developing cow's milk allergy. Partially hydrolysed proteins are compositions in which 60-70% of the protein/peptide population has a molecular weight of less than 1000 Daltons. Partially hydrolysed proteins are usually considered as hypoallergenic (HA). The milk protein hydrolysate may have an extent of hydrolysis that is characterised by NPN/TN %. Non-Protein Nitrogen over Total Nitrogen is widely use as a measure of soluble peptides created by enzymatic hydrolysis. NPN/TN% means the Non Protein Nitrogen divided by the Total Nitrogen X 100. NPN/TN% may be measured as detailed in Adler-Nisse n J-, 1979, J. Agric. Food Chem., 27 (6), 1256-1262. In general, partially hydrolysed proteins are characterized as having a NPN/TN% in the range 75%-85%. Preferably, the proteins hydrolysate has an NPN/TN % in the range of 70-90%, preferably 75 to 85%.

If hydrolysed proteins are required, the hydrolysis process may be carried out as desired and as is known in the art. For example, a whey protein hydrolysate may be prepared by enzymatically hydrolysing the whey fraction in one or more steps. If the whey fraction used as the starting material is substantially lactose free, it is found that the protein suffers much less lysine blockage during the hydrolysis process. This enables the extent of lysine blockage to be reduced from about 15% by weight of total lysine to less than about 10% by weight of lysine; for example about 7% by weight of lysine which greatly improves the nutritional quality of the protein source. Free arginine, free histidine, free taurine, free tyrosine, and either tryptophan rich milk protein, free tryptophan or a mixture thereof may be added. Nucleotides, as well as carnitine may be added in the infant formulas as a source of nitrogen too.

Hence, the source of proteins may comprise whey proteins, casein, hydrolysates thereof, and mixtures thereof.

Suitable carbohydrates source are lactose, saccharose, maltodextrin, starch. Mixtures thereof may be used. The source of carbohydrates comprises preferably lactose. Preferably, the source of carbohydrates comprises at least 80% lactose, more preferably at least 90% lactose, and even more preferably at least 95% lactore. In a preferred embodiment, the source of carbohydrates is exclusively lactose. Usually, when the infant formula comprises lactose, lactose is present in an amount ranging from 9 to 13 g/100kcal, preferably from 10 to 12 g/100kcal.

The infant formula generally contains a source of lipids. In this case, the lipid source may be any lipid or fat which is suitable for use in infant formulae. Preferred fat sources include milk fat, safflower oil, egg yolk lipid, canola oil, olive oil, coconut oil, palm kernel oil, soybean oil, fish oil, palm oleic, high oleic sunflower oil and high oleic safflower oil, and microbial fermentation oil containing long-chain, polyunsaturated fatty acids. The lipid source may also be in the form of fractions derived from these oils such as palm olein, medium chain triglycerides, and esters of fatty acids such as arachidonic acid, linoleic acid, palmitic acid, stearic acid, docosahexaeonic acid, linolenic acid, oleic acid, lauric acid, capric acid, caprylic acid, caproic acid, and the like. It may also be added small amounts of oils containing high quantities of preformed arachidonic acid and docosahexaenoic acid such as fish oils or microbial oils. The fat source preferably has a ratio of n-6 to n-3 fatty acids of about 5:1 to about 15:1; for example about 8:1 to about 10:1.

In an embodiment, the infant formula has an energy density comprised from 600 kcal/L to 780 kcal/L, preferably from 630 kcal/L to 700 kcal/L.

The infant formula also contains preferably all vitamins and minerals understood to be essential in the daily diet and in nutritionally significant amounts. Minimum requirements have been established for certain vitamins and minerals. Examples of minerals, vitamins and other nutrients optionally present in the infant formula include vitamin A, vitamin B1, vitamin B2, vitamin B6, vitamin B12, vitamin E, vitamin K, vitamin C, vitamin D, folic acid, inositol, niacin, biotin, pantothenic acid, choline, calcium, phosphorous, iodine, iron, magnesium, copper, zinc, manganese, chlorine, potassium, sodium, selenium, chromium, molybdenum, taurine, and L-carnitine. Minerals are usually added in salt form. The presence and amounts of specific minerals and other vitamins will vary depending on the intended population.

If necessary, the infant formula may contain emulsifiers and stabilisers such as soy, lecithin, citric acid esters of mono- and di-glycerides, and the like.

The infant formula may also contain at least one probiotic. Prefered probiotics are those which as a whole are safe, are L(+) lactic acid producing cultures and have acceptable shelf-life for products such as infant and follow-on formulae which are required to remain stable and effective for up to 36 months.

The infant formula has preferably a reduced level of electrolytes compared to standard infant and follow-on formula. For example, the NA/K ratio (mmol) may be around 0.4, the (Na+K)/Cl ratio (mmol) may be around 1.8, Na+K+Cl may be around 34 (mmol), and (Na+K)-Cl may be around 10 (mmol). The infant formula has preferably a low phosphate content, and preferably a phosphorus content of less than 40 mg/100kcal. Preferably, the infant formula has a calcium content between 35 and 45 g/100 mL, a phosphorus content between 15 and 25 mg/mL, and a Ca/P ratio is between 1.4 and 3.

The infant formula may also contain other substances which may have a beneficial effect such as lactoferrin, fibres, nucleotides, nucleosides, and the like.

All the uses stated above are particularly intended for infants and young children, particularly between 0 to 36 months of life. Particularly the infant formulas and uses are suited for infants at risk of cardiovascular diseases, in particular infants having a family history of cardiovascular diseases. More particularly the infant formulas and uses are suited for the infants at risk of cardiovascular diseases associated with high blood pressure, in other words, cardiovascular diseases where high blood pressure is one of their symptoms or causes.

The infant formula can be a starter infant formula, or a follow-on formula, and preferably the composition is a starter infant formula. The infant formula for use according to the invention is for consumption by an infant, as a liquid composition. Nevertheless, the infant formula can be provided as a powder infant formula which can be reconstituted in a liquid form by mixing the powder infant formula with water.

The dosage regimen of the infant formula may be devised based on three parameters that may be combined together, and which will be detailed below:
- the level of daily food intake represented by the infant formula,
- the duration of the regimen,
- the starting date of the regimen.

In this specification, food intake is assessed in terms of energy intake.

Preferably, the infant formula should represent at least 50% of the daily food (or energy) intake of the infant, more preferably at least 60%, or even at least 70%, of the daily food (or energy) intake of the infant. In another preferred embodiment, the infant formula represents at least 80% of the daily food (or energy) intake of the infant. Preferably, the infant formula represents the exclusive food (or energy) intake of the infant.

This regimen should preferably be maintained for at least three months, within a year from birth of the infant. The regimen may be maintained for more than three months, for instance for at least 6 months, and even as long as infant formula is an appropriate food for this age category. For instance, the regimen may be maintained from birth up to three to six months of age, or even up to 9 or 12 months of age. Preferably, the regimen begins at birth of the infant, including in the case of preterm infants, and ends within a year from birth. The regimen may evolve during the life of the infant. The infant formula may be a starter formula, during the first four to six months after birth, and may represent the exclusive food (or energy) intake of the infant during that period. Once food diversification begins, the infant formula may be a follow-on formula. It then represents a lower food (or energy) intake for the infant, for instance from 30%, 40% or 50%, up to 90%, 80% or 70% of the daily food (or energy) intake, depending on the amount of non-formula food given to the infant. Customarily, the amount of non-formula food increases over time.

For instance, the infant formula represents at least 50% of the daily food (or energy) intake during at least three months up to six months, and within a year from birth. Preferably, the infant formula represents at least 50% of the daily (or energy) food intake of the infant during the first three months up to the first six months of life of the infant, i.e. within three to six months from birth. Even more preferably, the infant formula represents at least 80% of the daily food (or energy) intake of the infant during at least the first three months up to the first six months of life of the infant. In yet another regimen, the infant formula represents the exclusive food (or energy) intake of the infant during at least the first three months of life of the infant.

The invention also relates to a method for preventing cardiovascular diseases in a patient, or to a method for preventing high blood pressure in a patient. Said methods comprise feeding a patient with an infant formula during at least three months and within a year after birth of the patient, wherein said infant formula comprises at least a source of proteins, a source of lipids, a source of carbohydrates, and wherein the protein content of the infant formula is below 2.1g/100kcal. The infant formula for use in these methods has been disclosed above, as well as several dosage regimens that may be implemented.

It should be noted here that specific regimen is followed by the patient at a very young age, preferably during three to six months from birth, up to 12 months of age. However, the expected preventive effect may be observed later in life of the patient, for instance as a young child, as a teenager, or as an adult.

In a preferred embodiment of the methods above, the infant formula represents at least 50% of the daily food (or energy) intake of the patient.

An infant formula which may be used according to the invention will now be described by way of example.

The formula may be prepared in any suitable manner. For example, it may be prepared by blending together the protein source, the carbohydrate source, and the fat source in appropriate proportions. If used, the emulsifiers may be included at this point. The vitamins and minerals may be added at this point but are usually added later to avoid thermal degradation. Any lipophilic vitamins, emulsifiers and the like may be dissolved into the fat source prior to blending. Water, preferably water which has been subjected to reverse osmosis, may then be mixed in to form a liquid mixture. The temperature of the water is conveniently in the range between about 50°C and about 80°C to aid dispersal of the ingredients. Commercially available liquefiers may be used to form the liquid mixture. The liquid mixture is then homogenised, for example in two stages.

The liquid mixture may then be thermally treated to reduce bacterial loads, by rapidly heating the liquid mixture to a temperature in the range between about 80°C and about 150°C for a duration between about 5 seconds and about 5 minutes, for example. This may be carried out by means of steam injection, an autoclave or a heat exchanger, for example a plate heat exchanger.

Then, the liquid mixture may be cooled to between about 60°C and about 85°C for example by flash cooling. The liquid mixture may then be again homogenised, for example in two stages between about 10 MPa and about 30 MPa in the first stage and between about 2 MPa and about 10 MPa in the second stage. The homogenised mixture may then be further cooled to add any heat sensitive components, such as vitamins and minerals. The pH and solids content of the homogenised mixture are conveniently adjusted at this point.

The homogenised mixture is transferred to a suitable drying apparatus such as a spray dryer or freeze dryer and converted to powder. The powder should have a moisture content of less than about 5% by weight.

If a liquid infant formula is preferred, the homogenised mixture may be sterilised then aseptically filled into suitable containers or may be first filled into the containers and then retorted.

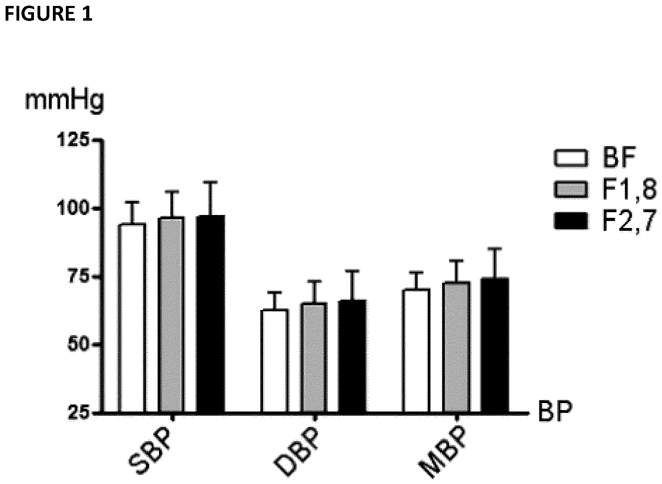

The techniques of the present invention will be readily understood by considering the accompanying drawing, Figure 1, which is a diagram showing the measurements of the blood pressure (BP) (Systolic Blood Pressure, SDP; Diastolic Blood Pressure, DBP; Mean Blood Pressure, MBP), with respect to the use of three different feeding infant formulas, (Breastfeeding, BF; infant formula for use according to the invention, F1.8; comparative infant formula F. 2.8).

Figure 1 will be explained in the following examples.

The invention is further illustrated by the following non-limiting examples which are given for illustrative purposes only.

### EXAMPLES

A composition of an infant formula for use according to the present invention, named F1.8, is given in Table 1 below (by way of illustration only).

**Table 1**

| Nutrient | per 100kcal | per litre |
|---|---|---|
| Energy (kcal) | 100 | 670 |
| Protein (g) | 1.83 | 12.3 |
| Fat (g) | 5.3 | 35.7 |
| Linoleic acid (g) | 0.79 | 5.3 |
| α-Linolenic acid (mg) | 101 | 675 |
| Lactose (g) | 11.2 | 74.7 |
| Minerals (g) | 0.37 | 2.5 |
| Na (mg) | 23 | 150 |
| K (mg) | 89 | 590 |
| Cl (mg) | 64 | 430 |
| Ca (mg) | 62 | 410 |
| P (mg) | 31 | 210 |
| Mg (mg) | 7 | 50 |
| Mn (µg) | 8 | 50 |
| Se (µg) | 2 | 13 |
| Vitamin A (µg RE) | 105 | 700 |
| Vitamin D (µg) | 1.5 | 10 |
| Vitamin E (mg TE) | 0.8 | 5.4 |
| Vitamin K1 (µg) | 8 | 54 |
| Vitamin C (mg) | 10 | 67 |
| Vitamin B1 (mg) | 0.07 | 0.47 |
| Vitamin B2 (mg) | 0.15 | 1.0 |
| Niacin (mg) | 1 | 6.7 |
| Vitamin B6 (mg) | 0.075 | 0.50 |
| Folic acid (µg) | 9 | 60 |
| Pantothenic acid (mg) | 0.45 | 3 |
| Vitamin B12 (µg) | 0.3 | 2 |
| Biotin (µg) | 2.2 | 15 |
| Choline (mg) | 10 | 67 |
| Fe (mg) | 1.2 | 8 |
| I (µg) | 15 | 100 |
| Cu (mg) | 0.06 | 0.4 |
| Zn (mg) | 0.75 | 5 |

A comparative study was conducted with a formula containing 2.7 g/ 100 kcal of proteins named F2.7. Both infant formulas F1.8 and F2.7 have a Energy of 670 kcal/L. The infant formulas F1.8 and F2.7 are different only with respect to the components in the Table 2 below:

**Table 2**

| **Nutrients \ Composition per 100 kCal** | **F1.8** | **F2.7** |
|---|---|---|
| Carbohydrates (Lactose) (g) | 11.16 | 10.38 |
| Protein (Casein/Whey: 30/70) (g) | 1.83 | 2.70 |
| Sodium (mg) | 23 | 26 |

The carbohydrates level was adjusted to ensure the same level of energy intake in both formulas F1.8 and F2.7. It is assumed that the difference of sodium content is linked to the difference in protein content. The effect of the different sodium content is considered negligible.

In a randomized study, the inventors have measured diastolic, systolic and mean blood pressure from 3 years old children who were exclusively fed during their first four months of life either:
- breastfed (BF) (n= 84) ; or
- with the low protein formula (1.8 g protein/100kcal) F1.8 for use according to the invention (n = 74); or
- with the comparative high protein formula (2.7 g protein/100kcal) F2.8 (n = 80).

Both breastfed and formula-fed infants could start complementary feeding at 4 months. The formula-fed infants were maintained on their respective formulas until 1 year of age and the breastfed group received the low protein formula F1.8. Formula could represent from 30 to 90% of the food intake of the infants, depending for instance on their age.

The results are summarized in the Table 3 below, and shown in Figure 1 which shows the measurements of the blood pressure (BP), that is to say Systolic Blood Pressure, SBP; Diastolic Blood Pressure, DBP; Mean Blood Pressure, MBP, with respect to the use of the three different infant formulas (Breastfeed, BF; infant formula for use according to the invention, F1.8; comparative infant formula F. 2.8).

**Table 3**

| Blood pressure (mm Hg) | Systolic SBP | Diastolic DBP | Mean blood pressure MBP |
|---|---|---|---|
| Breastfed | 94.04 ± 7.83 | 62.45 ± 6.56 | 69.72 ± 6.55 |
| 1.8 g protein/100kcal | 96.40 ± 9.31 | 64.84 ± 8.16 | 72.18 ± 8.05 |
| 2.7 g protein/100kcal | 96.70 ± 12.53 | 65.98 ± 10.67 | 74.05 ± 11.01 |

For systolic pressure (SBP), F 1.8 vs BF, p = 0.1849 ; F2.7 vs BF p = 0.1377 For diastolic pressure (DBP), F 1.8 vs BF, p = 0.0798 ; F2.7 vs BF p<0.04 For mean blood pressure (MBP), F 1.8 vs BF, p = 0.0735 ; F2.7 vs BF p<0.01

A p-value lower than 0.05 means that the difference is significant.

Thus the inventors have shown that 3-year old children exclusively fed with a low protein formula (1.8 g protein/100kcal) during at least their first 4 months of life had a blood pressure closer to children who were exclusively breastfed than those exclusively fed a high protein formula (2.7 g protein/100kcal).

### BIBLIOGRAPHY

Boubred et al. (2007). Effects of early postnatal hypernutrition on nephron number and long-term renal function and structure in rats. Am J Physiol Renal Physiol, 293 : 1944-1949.
Jarvisalo et al. (2009). Breast feeding in infancy and arterial endothelial function later in life. European journal of clinical nutrition, 63(5) : 640-645

## Claims

1. An infant formula comprising at least a source of proteins, a source of lipids, a source of carbohydrates, wherein the protein content is within the range of 1.6 to 1.85g/100kcal, wherein the proteins are hydrolysed, wherein the source of proteins is a mixture of whey proteins and casein, and the whey/casein ratio is at least equal to 50/50, wherein the major part of caseino-glycomacropeptide (CGMP) has been removed from whey proteins, for administration to the infant exclusively during at least its first 4 months of life and for at least 50% of the daily food intake within three to six months from birth, for use in the prevention of high blood pressure or cardiovascular diseases associated with high blood pressure.

2. The formula for use according to claim 1, wherein the proteins are selected from milk proteins, milk protein partial hydrolysates, hypoallergenic milk protein hydrolysates, and mixtures thereof.

3. The formula for use according to any one of claims 1 and 2, wherein the source of proteins comprises whey proteins, casein, hydrolysates thereof, or mixtures thereof.

4. The formula for use according to any one of claims 1 to 3, wherein the source of carbohydrates comprises lactose.

5. The formula for use according to claim 4, wherein the content of lactose ranges from 9 to 13g/100kcal.

6. The formula for use according to any one of claims 1 to 5, for administration to the infant for at least 50% of the daily food intake during at least three months and within a year from birth.

## Patentansprüche

1. Säuglingsnahrung, umfassend mindestens eine Proteinquelle, eine Lipidquelle, eine Kohlenhydratquelle, wobei der Proteingehalt im Bereich von 1,6 bis 1,85 g/418,4 kJ (100 kcal) liegt, wobei die Proteine hydrolysiert sind, wobei die Proteinquelle ein Gemisch aus Molkenprotein und Casein ist und das Molke-/Casein-Verhältnis mindestens 50/50 entspricht, wobei der größte Teil des Casein-Glykomakropeptids (CGMP) aus den Molkenproteinen entfernt wurde, zur Verabreichung an den Säugling ausschließlich während mindestens seiner ersten 4 Lebensmonate und als mindestens 50 % der täglichen Nahrungsaufnahme innerhalb von drei bis sechs Monaten ab der Geburt, zur Verwendung in der Vorbeugung von hohem Blutdruck oder kardiovaskulären Erkrankungen, die mit hohem Blutdruck in Verbindung gebracht werden.

2. Formel zur Verwendung nach Anspruch 1, wobei die Proteine ausgewählt werden aus Milchproteinen, Milchprotein-Teilhydrolysaten, hypoallergenen Milchprotein-Hydrolysaten und Gemischen davon.

3. Formel zur Verwendung nach einem der Ansprüche 1 und 2, wobei die Proteinquelle Molkenproteine, Casein, Hydrolysate davon oder Gemische davon umfasst.

4. Formel zur Verwendung nach einem der Ansprüche 1 bis 3, wobei die Kohlenhydratquelle Laktose umfasst.

5. Formel zur Verwendung nach Anspruch 4, wobei der Laktosegehalt im Bereich zwischen 9 und 13 g/418,4 kJ (100 kcal) liegt.

6. Formel zur Verwendung nach einem der Ansprüche 1 bis 5 zur Verabreichung an den Säugling als mindestens 50 % der täglichen Nahrungsaufnahme über mindestens drei Monate und innerhalb eines Jahres ab der Geburt.

## Revendications

1. Préparation pour nourrissons comprenant au moins une source de protéines, une source de lipides, une source de glucides, dans laquelle la teneur en protéines se situe dans l'intervalle de 1,6 à 1,85 g/100 kcal, dans laquelle les protéines sont hydrolysées, dans laquelle la source de protéines est un mélange de protéines lactosériques et de caséine et le rapport lactosérum/caséine est au moins égal à 50/50, dans laquelle la majeure partie de caséino-glycomacropeptide (CGMP) a été retirée des protéines lactosériques, pour administration au nourrisson exclusivement pendant au moins les 4 premiers mois de sa vie et à raison d'au moins 50 % de l'apport alimentaire quotidien dans les trois à six mois suivant la naissance, pour une utilisation dans la prévention d'une pression sanguine élevée ou de maladies cardiovasculaires associées à une pression sanguine élevée.

2. Préparation utilisable selon la revendication 1, dans laquelle les protéines sont choisies parmi des protéines laitières, des hydrolysats partiels de protéines laitières, des hydrolysats de protéines laitières hypoallergéniques et leurs mélanges.

3. Préparation utilisable selon l'une quelconque des revendications 1 et 2, dans laquelle la source de protéines comprend des protéines lactosériques, de la caséine, des hydrolysats de celles-ci ou des mélanges des celles-ci.

4. Préparation utilisable selon l'une quelconque des revendications 1 à 3, dans laquelle la source de glucides comprend du lactose.

5. Préparation utilisable selon la revendication 4, dans laquelle la teneur en lactose va de 9 à 13 g/100 kcal.

6. Préparation utilisable selon l'une quelconque des revendications 1 à 5, pour administration au nourrisson à raison d'au moins 50 % de l'apport alimentaire quotidien pendant au moins trois mois et dans l'année suivant la naissance.
